# EUROPEAN PATENT SPECIFICATION

(11) **EP 3 534 874 B1**
(45) Date of publication and mention of the grant of the patent: **24.02.2021**
(21) Application number: 17811377.5
(22) Date of filing: 27.10.2017
(51) Int. Cl.: A61K 8/34, A61K 8/49, A61K 8/60, A61Q 19/08, A61K 8/88, A61K 8/22

(54) **COSMETIC METHOD**
KOSMETISCHES VERFAHREN
PROCÉDÉ COSMÉTIQUE

(30) Priority: 04.11.2016 IT 201600111275
(43) Date of publication of application: 11.09.2019
(73) Proprietor: Centro Dermatologico S.r.l., 40138 Bologna (IT)
(72) Inventor: NEGOSANTI, Francesca, 40137 Bologna (IT)
(74) Representative: Biggi, Cristina
(86) International application number: PCT/IB2017/056683
(87) International publication number: WO 2018/083574

(56) References cited:
- US-A- 4 956 173
- US-A- 5 626 849
- US-A1- 2004 047 896
- Anonymous: "Introducing Ozone Therapy - A powerful Anti--Ageing Treatment", , 4 October 2013 (2013-10-04), XP055395565, Retrieved from the Internet: URL:http://www.dotolo.eu/blog/wp-content/u ploads/2013/09/ozone-seminar-flyer.pdf [retrieved on 2017-08-02]
- Anonymous: "A colon cleanse a year can lead to successful aging!", , 2 August 2017 (2017-08-02), XP055395561, Retrieved from the Internet: URL:http://www.best-anti-aging-secrets.com /colon-cleanse.html [retrieved on 2017-08-02]

## Description

The object of the present invention is a cosmetic method for the treatment and/or prevention of the signs of aging in a human being.

### State of the art

Antiaging is a philosophy that is now very widespread throughout the world and that has the objective of helping those who are well to constantly improve their wellness and the objective of preventing the physical signs of aging in a person as much as possible.

Aging is a completely natural process. However, the changes imposed by this process at the aesthetic level are often unsettling for the individual, who may end up perceiving the aging process as personal and social failure.

A healthy lifestyle, good nutrition and adequate physical exercise surely represent the best method for preventing early aging of the mind and body.

However, targeted cosmetic treatments can aid in rapidly and effectively reaching the objectives of aesthetic improvement that individuals set for themselves, acting as an accelerator in a plan for modifying one's customary lifestyle.

The traditional approach to the treatment and/or prevention of the signs of aging considers separately the exterior signs of aging - the treatment or prevention of which is a prerogative of cosmetics - and the signs of aging that appear in the rest of the body, such as neurodegenerative diseases or tumours, whose treatment is instead an exclusive prerogative of the medical field.

Traditional cosmetic treatments for preventing and/or attentuating aging of the skin generally involve the use of products or technologies for topical skin treatment, for example with antiwrinkle, toning, moisturizing and other effects.

The most modern antiaging philosophy is based on a holistic approach to the individual, considering the human being as a complex system, in which even a cosmetic treatment for the prevention and/or reduction of the signs of aging cannot but involve the entirety of the individual and not just one part of the individual (e.g. the epidermis or the dermis).

In this context, the present invention has the objective of making available a non-therapeutic cosmetic method for the prevention and/or reduction of the signs of aging, based on a synergistic approach of the various steps constituting the treatment itself.

### Summary of the invention

The present invention refers to a non-therapeutic cosmetic method for treating and/or preventing the signs of aging in a human being, comprising at least one step of colon cleansing, at least one step of administering ozone, at least one step of administering an effective dose of a compound selected from among inositol and ademetionine, wherein ademetionine is administered once every 5-15 days, preferably once every 7-10 days, and at least one step of administering a compound selected from among glutathione, melatonin and mixtures thereof.

### Detailed description

The non-therapeutic cosmetic method according to the present invention is carried out on healthy human beings, including both males and females without distinction, particularly on human beings who do not have diseases affecting the intestinal tract, including for example diverticulitis, cancer, chronic inflammatory diseases affecting the intestines, etc.

In this description and in the appended claims, the term "healthy human being" refers to a human being without diseases affecting the intestinal tract, particularly the diseases listed hereinabove.

The presence of diseases affecting the intestinal tract can be excluded by preliminarily subjecting the individual to appropriate blood chemistry tests, as well as laboratory tests to check for the existence of any conditions associated with intestinal dysbiosis.

The cosmetic treatment according to the invention comprises at least one step of colon cleansing. The colon cleansing step is carried out by having water, preferably filtered water from the water supply, flow into the intestines through the use of suitable equipment: through a first rectal cannula, the water is introduced into the intestines, from which it is eliminated, together with faecal matter removed from the intestines, through a second cannula. Water temperature and pressure are kept stable during the treatment. Water temperature can be adjusted to values of about 25-41 °C, preferably to about 37° C.

Each single step of colon cleansing can be of a duration of about 20-60 minutes, preferably of about 30-45 minutes.

According to a preferred form of implementation, the at least one colon cleansing step can be carried out once every 7-21 days, preferably once every 12-17 days, more preferably once every 15 days.

The step of colon cleansing enables removal of residual faecal matter from the intestines, performs a beneficial intestinal massage and helps the colon to reassume its natural form, producing a sensation of well-being in the subject.

The cosmetic method according to the invention further comprises at least one step of administering ozone to the subject (exposing the subject to ozone). The aim of exposing the subject to ozone is to decrease the free radicals present in the subject's body, thereby reducing oxidative stress. Ozone can be administered through a route of administration selected from among intravenous, transrectal, nasal, or intravaginal-vulval-ureteral administration, preferably it can be administered intravenously, using techniques and equipment already known per se in the prior art.

The administration of ozone through one of the routes indicated above is of a nonsurgical nature, for it does not represent a substantial procedure carried out on the human body. The health risk associated with the administration of ozone through the routes indicated above is minimal when the administration process is carried out with due care by professionally trained staff.

According to one preferred form of implementation, the at least one step of administering ozone can be carried out by administering ozone-enriched (pretreated) blood to the subject. Pretreatment of the blood is carried out through suitable equipment operating under sterile conditions so as to prevent the risk of contamination during the ozone-enrichment process. The blood can preferably be an isolated blood sample collected previously from the same subject undergoing the cosmetic treatment.

The equipment used to administer ozone, is already known per se to the person skilled in the art, as are the operating conditions used for carrying out this step of the treatment (duration, amount of ozone administered, etc.).

Preferably, the at least one step of administering ozone can be carried out once every 3-15 days, preferably every 5-10 days, more preferably every 7 days.

In a further aspect, the present invention can refer to a non-therapeutic cosmetic method for treating and/or preventing the signs of aging in a human being to whom ozone has been pre-administered, wherein said method comprises at least one step of colon cleansing, at least one step of administering an effective dose of a compound selected from among inositol and ademetionine, wherein ademetionine is administered once every 5-15 days, preferably once every 7-10 days, and at least one step of administering a compound selected from among glutathione, melatonin and mixtures thereof, said steps being as described herein.

The cosmetic method according to the invention further comprises at least one step of administering an effective dose of a compound selected from among inositol and ademetionine, wherein the administration of ademetionine takes place once every 7-15 days, preferably once 7-10 days.

Inositol, whose formula is C₆H₁₂O₆, is a polyol of cyclohexane with six -OH groups. It can exist in nine stereoisomers, of which myo-inositol (cis-1,2,3,5-trans-4,6-cyclohexanehexol) is the most widely occurring form in nature and the form that can be used in the cosmetic method of the invention.

Inositol can be administered in a single dose of about 1000-6000 mg, preferably in a single dose of about 1000 mg. The route of administration for inositol can be oral, intramuscular or intravenous, preferably intravenous.

According to a preferred form of implementation, inositol can be administered to the subject once every 7-15 days, preferably once every 7-10 days, preferably about every 7 days.

Ademetionine, or S-Adenosyl metionine is an active ingredient that is used for treating depressive disorders. It is known that to treat depressive disorders, 100-400 mg/day of ademetionine should be administered intravenously, or 800-1200 mg/day orally, for at least 20 days. It is also known that the active ingredient begins to take effect on the symptoms associated with the depressive disorder about 5-7 days after starting the continuative treatment. Therefore, the cosmetic method of the invention is not of a therapeutic nature, for ademetionine is administered to the subject once every 5-15 days, preferably once every 7-10 days.

In fact, the Applicant has surprisingly found that while there is no strictly therapeutic effect, the administration of inositol or ademetionine in the single doses reported above contributes to reducing the environmental stress levels in a subject and, in synergy with the further steps of the treatment method described herein, to attenuating oxidative stress.

Ademetionine can be administered in a single dose of about 100-400 mg, preferably in a single dose of about 200 mg. The route of administration for ademetionine can be selected from among intravenous, intramuscular and oral routes, preferably an intravenous route of administration.

The administration of inositol or ademetionine through one of the routes indicated above is of a nonsurgical nature, for it does not represent a substantial procedure carried out on the human body. The health risk associated with the administration of inositol or metionine through the routes indicated above is minimal when the administration process is carried out with due care by professionally trained staff.

The cosmetic treatment method further comprises at least one step of administering a compound selected from among glutathione, melatonin and mixtures thereof.

The administration of a compound selected from among glutathione, melatonin or mixtures thereof can preferably take place by oral administration. Preferably, said compounds can be administered in the following single doses of:
- about 100-500 mg of glutathione, preferably about 250 mg;
- about 0.5 - 1.5 mg of melatonin, preferably about 1 mg.

Glutathione can be administered as glutathione and/or reduced glutathione.

According to one form of implementation, glutathione and melatonin can be administered during this step of the treatment.

According to one form of implementation, this step of the treatment can be carried out once a day.

According to one form of implementation, the cosmetic method according to the invention can comprise at least one further step of administering at least one of the following: vitamin E, alpha-tocopheryl acetate, retinol, trans-retinoic acid, beta-carotene, dehydroepiandrosterone, methyl sulfonyl methane, glucosamine, chondroitin, tomato extract, grape extract, turmeric, tocotrienol, polyunsaturated fatty acids, vitamin C and mixtures thereof.

The administration of these compounds has a synergistic effect with the other steps of the treatment, proving to be effective in decreasing the level of free radicals, acting positively on the signs of aging principally due to high levels of oxidative stress.

According to one form of implementation, the polyunsaturated fatty acids can comprise alpha-linoleic acid, eicosapentaenoic acid, docosahexanoic acid and mixtures thereof.

In this further step, the administration of the compounds indicated above can preferably take place by oral administration, in a daily effective dose preferably of:
- about 10-20 g/day (total), preferably about 15 g/day (total) of vitamin E and/or alpha-tocopheryl acetate;
- about 10-30 mg/day (total) of retinol and/or trans-retinoic acid;
- about 20-150 mg/day of beta-carotene;
- about 30-70 mg/day, preferably about 50 mg/day of dehydroepiandrosterone;
- about 300-700 mg/day, preferably about 500 mg/day of methyl sulfonyl methane;
- about 300-700 mg/day, preferably about 500 mg/day of glucosamine;
- about 300-500 mg/day, preferably about 400 mg/day of chondroitin;
- about 30-50 mg/day, preferably about 40 mg/day, of tomato extract, preferably dry extract, lycopene,
- about 15-30 mg/day, preferably about 20 mg/day, of grape extract, preferably dry extract.
- about 1000 - 2000 mg/day, preferably about 1600 mg/day of turmeric;
- about 50 - 150 mg/day, preferably about 100 mg/day of tocotrienol;
- about 50-400 mg/day (total) of polyunsaturated fatty acids.

According to one form of implementation, administration can include about 20-100 mg/day, preferably about 70 mg/day of alpha-linolenic acid; about 30-150 mg/day, preferably about 100 mg/day of eicosapentanoic acid; about 30-150 mg/day, preferably about 100 mg/day of docosahexanoic acid and mixtures thereof;
- about 100-200 mg/day, preferably about 120-150 mg/day of vitamin C.

The compounds indicated above can be administered individually or, and preferably, in the form of compositions comprising two or more compounds.

The cosmetic method according to the invention can be carried out once only or, and preferably, repeated consecutively for a number of times ≥ 2, preferably 2-6 consecutive times.

According to a preferred form of implementation, the cosmetic method of the invention can be carried out repeatedly for an overall duration of about two months.

The various steps constituting the cosmetic treatment according to the invention can be carried out simultaneously or consecutively in any order. The cosmetic treatment method of the invention has proved to be effective in treating and/or preventing the signs of aging in a human being, reducing oxidative stress factors related to environmental stress, to improper diets, and excessive lifestyles (smoking, alcohol consumption, UV radiation exposure). Upon completion of the treatment, the subject perceives an improvement in his/her aesthetic appearance, which means feeling and seeing oneself as younger.

This improvement can be objectively quantified by monitoring the change in several parameters related to the signs of aging before and after treatment, as described herein below.

Moreover, the cosmetic method of the invention can be enhanced by including it in a treatment protocol that also comprises a proper diet and adequate physical activity carried out under the supervision of skilled personnel.

### Example

To demonstrate the effectiveness of the cosmetic treatment method according to the invention, we tested the method on 20 subjects (10 women and 10 men), all of whom were healthy subjects.

The subjects were considered to be suitable for treatment after having examined their overall health status and excluded the presence of intestinal diseases through a set of blood chemistry tests, urinalyses and tests to check for the presence of any situations associated with intestinal dysbiosis.

The subjects underwent the cosmetic treatment according to the following protocol for an overall duration of two months:
- administration of ozone intravenously: once every 7 days;
- colon cleansing for the duration of 40 minutes: once every 15 days;
- administration of glutathione: 250 mg/day
- administration of melatonin: 1 mg/day

Moreover, 1000 mg of inositol were administered to 10 subjects intravenously once every 7 days (subjects called the "inositol group") and 200 mg of ademetionine were administered to 10 subjects every 7 days intravenously (subjects called the "ademetionine group").

The effectiveness of the cosmetic method was assessed according to a subjective method through interviews with the subjects who had undergone the treatment method. All the treated subjects stated that they had observed an improvement in their aesthetic appearance.

Furthermore, the effectiveness of the method was objectively assessed by measuring the oxidative stress levels and the antioxidant potential through the FRAS5 system by H&D Sri, before and at the end of the two months of treatment.

All subjects showed a decrease in oxidative stress levels and an increase in the antioxidant potential.

## Claims

1. A non-therapeutic cosmetic method for treating and/or preventing the signs of aging in a human being, comprising at least one step of colon cleansing, at least one step of administering ozone, at least one step of administering an effective dose of at least one compound selected from among inositol and ademetionine, wherein ademetionine is administered once every 5-15 days, preferably once every 7-10 days, and at least one step of administering a compound selected from among glutathione, melatonin and mixtures thereof.

2. The non-therapeutic cosmetic method according to claim 1, further comprising at least one step of administering at least one from among vitamin E, alpha-tocopheryl acetate, retinol, trans-retinoic acid, beta-carotene, dehydroepiandrosterone, methyl sulfonyl methane, glucosamine, chondroitin, tomato extract, grape extract, turmeric, tocotrienol, polyunsaturated fatty acids, vitamin C and mixtures thereof.

3. The non-therapeutic cosmetic method according to claim 1 or 2, wherein inositol is administered once every 5-15 days, preferably once every 7-10 days, more preferably once every 7 days.

4. The non-therapeutic cosmetic method according to any one of the preceding claims, wherein inositol is administered in a single dose of 1000-6000 mg/, preferably a single dose of 1000 mg.

5. The non-therapeutic cosmetic method according to any one of the preceding claims, wherein ademetionine is administered in a single dose of 100-400 mg, preferably a single dose of 200 mg.

6. The non-therapeutic cosmetic method according to any one of the preceding claims, wherein the step of colon cleansing is carried out once every 7-21 days, preferably once every 12-17 days, more preferably once every 15 days.

7. The non-therapeutic cosmetic method according to any one of the preceding claims, wherein the step of administering ozone is carried out once every 3-15 days, preferably once every 5-10 days, more preferably once every 7 days.

8. The non-therapeutic cosmetic method according to any one of the preceding claims, comprising administering glutathione in a single dose of 100-500 mg, preferably a single dose of 250 mg, melatonin in a single dose of 0.5 - 1.5 mg, preferably a single dose of 1.0 mg, and mixtures thereof.

9. The non-therapeutic cosmetic method according to any one of the preceding claims, comprising administering glutathione and melatonin, preferably once a day.

## Patentansprüche

1. Nichttherapeutisches kosmetisches Verfahren zur Behandlung und/oder Verbeugung der Anzeichen der Alterung in einem Menschen, umfassend mindestens einen Schritt zur Darmreinigung, mindestens einen Schritt zur Verabreichung von Ozon, mindestens einen Schritt zur Verabreichung einer wirksamen Dosis von mindestens einer Verbindung, die aus Inosit und Ademetionin ausgewählt ist, wobei Ademetionin einmal alle 5-15 Tage, vorzugsweise einmal alle 7-10 Tage, verabreicht wird, und mindestens einen Schritt zur Verabreichung einer Verbindung, die aus Glutathion, Melatonin und Gemischen davon ausgewählt ist.

2. Nichttherapeutisches kosmetisches Verfahren nach Anspruch 1, ferner umfassend mindestens einen Schritt zur Verabreichung von mindestens einem aus Vitamin E, Alpha-Tocopherylacetat, Retinol, Trans-Retinoinsäure, Beta-Carotin, Dehydroepiandrosteron, Methylsulfonylmethan, Glucosamin, Chondroitin, Tomatenextrakt, Traubenextrakt, Kurkuma, Tocotrienol, mehrfach ungesättigten Fettsäuren, Vitamin C und Gemischen davon.

3. Nichttherapeutisches kosmetisches Verfahren nach Anspruch 1 oder 2, wobei Inosit einmal alle 5-15 Tage, vorzugsweise einmal alle 7-10 Tage, bevorzugter einmal alle 7 Tage verabreicht wird.

4. Nichttherapeutisches kosmetisches Verfahren nach einem der vorhergehenden Ansprüche, wobei Inosit in einer Einzeldosis von 1000-6000 mg, vorzugsweise einer Einzeldosis von 1000 mg verabreicht wird.

5. Nichttherapeutisches kosmetisches Verfahren nach einem der vorhergehenden Ansprüche, wobei Ademetionin in einer Einzeldosis von 100-400 mg, vorzugsweise einer Einzeldosis von 200 mg, verabreicht wird.

6. Nichttherapeutisches kosmetisches Verfahren nach einem der vorhergehenden Ansprüche, wobei der Schritt zur Darmreinigung einmal alle 7-21 Tage, vorzugsweise einmal alle 12-17 Tage, bevorzugter einmal alle 15 Tage durchgeführt wird.

7. Nichttherapeutisches kosmetisches Verfahren nach einem der vorhergehenden Ansprüche, wobei der Schritt zur Verabreichung von Ozon einmal alle 3-15 Tage, vorzugsweise einmal alle 5-10 Tage, bevorzugter einmal alle 7 Tage durchgeführt wird.

8. Nichttherapeutisches kosmetisches Verfahren nach einem der vorhergehenden Ansprüche, umfassend die Verabreichung von Glutathion in einer Einzeldosis von 100-500 mg, vorzugsweise einer Einzeldosis von 250 mg, Melatonin in einer Einzeldosis von 0,5-1,5 mg, vorzugsweise einer Einzeldosis von 1,0 mg und Gemische davon.

9. Nichttherapeutisches kosmetisches Verfahren nach einem der vorhergehenden Ansprüche, umfassend die Verabreichung von Glutathion und Melatonin, vorzugsweise einmal am Tag.

## Revendications

1. Procédé cosmétique non thérapeutique pour traiter et/ou prévenir les signes du vieillissement chez un être humain, comprenant au moins une étape de nettoyage du côlon, au moins une étape d'administration d'ozone, au moins une étape d'administration d'une dose efficace d'au moins un composé choisi parmi l'inositol et l'adémétionine, dans lequel l'adémétionine est administrée une fois tous les 5-15 jours, de préférence une fois tous les 7-10 jours, et au moins une étape d'administration d'un composé choisi parmi le glutathion, la mélatonine et leurs mélanges.

2. Procédé cosmétique non thérapeutique selon la revendication 1, comprenant en outre au moins une étape d'administration d'au moins une substance parmi la vitamine E, l'acétate d'alpha-tocophéryle, le rétinol, l'acide transrétinoïque, le bêta-carotène, la déhydroépiandrostérone, le méthylsulfonylméthane, la glucosamine, la chondroïtine, l'extrait de tomate, l'extrait de raisin, le curcuma, le tocotriénol, les acides gras polyinsaturés, la vitamine C et leurs mélanges.

3. Procédé cosmétique non thérapeutique selon la revendication 1 ou 2, dans lequel l'inositol est administré une fois tous les 5-15 jours, de préférence une fois tous les 7-10 jours, plus préférablement une fois tous les 7 jours.

4. Procédé cosmétique non thérapeutique selon l'une quelconque des revendications précédentes, dans laquelle l'inositol est administré en une seule dose de 1000-6000 mg de préférence une seule dose de 1000 mg.

5. Procédé cosmétique non thérapeutique selon l'une quelconque des revendications précédentes, dans laquelle l'adémétionine est administrée en une seule dose de 100-400 mg, de préférence une seule dose de 200 mg.

6. Procédé cosmétique non thérapeutique selon l'une quelconque des revendications précédentes, dans laquelle l'étape de nettoyage du côlon est effectuée une fois tous les 7-21 jours, de préférence une fois tous les 12-17 jours, plus préférablement une fois tous les 15 jours.

7. Procédé cosmétique non thérapeutique selon l'une quelconque des revendications précédentes, dans lequel l'étape d'administration d'ozone est effectuée une fois tous les 3-15 jours, de préférence une fois tous les 5-10 jours, plus préférablement une fois tous les 7 jours.

8. Procédé cosmétique non thérapeutique selon l'une quelconque des revendications précédentes, comprenant l'administration de glutathion en une seule dose de 100-500 mg, de préférence une seule dose de 250 mg, de mélatonine en une seule dose de 0,5 - 1,5 mg, de préférence une seule dose de 1,0 mg, et de leurs mélanges.

9. Procédé cosmétique non thérapeutique selon l'une quelconque des revendications précédentes, comprenant l'administration de glutathion et de mélatonine, de préférence une fois par jour.
